# EUROPÄISCHE PATENTSCHRIFT

(11) **EP 3 946 575 B1**
(45) Veröffentlichungstag und Bekanntmachung des Hinweises auf die Patenterteilung: **13.03.2024**
(21) Anmeldenummer: 20718808.7
(22) Anmeldetag: 02.04.2020
(51) Int. Cl.: A61N 5/06, A61B 5/00, G06F 3/16, A61H 9/00, G10L 15/00, G10L 15/02

(54) **VORRICHTUNG ZUM EINWIRKEN AUF ZUMINDEST TEILE EINES KÖRPERS**
DEVICE FOR ACTING ON AT LEAST PARTS OF A BODY
DISPOSITIF POUR AGIR SUR AU MOINS DES PARTIES D'UN CORPS

(30) Priorität: 03.04.2019 CH 4552019
(43) Veröffentlichungstag der Anmeldung: 09.02.2022
(73) Patentinhaber: JK-Holding GmbH, 53578 Windhagen (DE)
(72) Erfinder: GERSTENMEIER, Jürgen, 56567 Neuwied (DE)
(74) Vertreter: IPrime Rentsch Kaelin AG
(86) Internationale Anmeldenummer: PCT/IB2020/053145
(87) Internationale Veröffentlichungsnummer: WO 2020/202062

(56) Entgegenhaltungen:
- CN-A- 108 743 204
- CN-A- 108 937 932
- CN-A- 108 969 897
- DE-A1-102012 007 504
- DE-C1- 4 431 402
- DE-U1-202018 105 021
- JP-A- 2005 000 376
- US-A1- 2009 031 491
- US-A1- 2013 096 473
- US-A1- 2014 350 411
- US-A1- 2016 008 568
- US-A1- 2017 157 431
- US-A1- 2017 160 710
- US-A1- 2017 238 401
- US-A1- 2017 361 125
- US-A1- 2018 014 777
- US-A1- 2018 368 762
- US-A2- 2015 174 003

## Beschreibung

Die Erfindung betrifft eine Vorrichtung zum Einwirken auf zumindest Teile eines Körpers eines Benutzers, insbesondere mit medizinisch-kosmetischer Strahlung und/oder durch mechanische Beeinflussung.

Aus der Praxis sind Vorrichtungen zum Einwirken auf Teile eines Körpers bekannt, bei denen aus regulatorischen oder aus Entspannungsgründen der Gebrauch der Augen nicht möglich ist. Beispielsweise sind Vorrichtungen zur Einwirkung auf den Körper eines Benutzers mittels Hautbestrahlung, insbesondere mit ultraviolettem Licht, nur mit Schutzbrille oder zumindest mit geschlossenen Augen zu benutzen, da die Strahlung in bestimmten Wellenlängen für die Augen schädlich sein kann. Nachteilig bei den bekannten Vorrichtungen ist der Umstand, dass individuelle Anpassungen an Wünsche oder Vorlieben der Benutzer über ein berührungsempfindliches Display erfolgen, das mit Schutzbrille praktisch kaum zu erkennen ist.

Im Bereich der Bestrahlung der Haut werden medizinisch-kosmetische Strahlung emittierende Anordnungen eingesetzt, deren Strahlung eine photobiologische Wirkung bei einer bestrahlten Person erzeugt. Die medizinisch-kosmetische Strahlung trifft hierbei auf die Haut der Person auf, kann aber je nach konkreter Wellenlänge in tiefere Regionen des Körpers eindringen. Die Wirkung umfasst beispielsweise eine Bräunung der Haut, aber auch weitere physiologische und psychologische Effekte resultieren aus der Bestrahlung. Medizinisch-kosmetische Strahlung umfasst das Spektrum der ultravioletten (UV-) Strahlung, der sichtbaren (VIS-) Strahlung und der nahen Infrarot (nIR-) Strahlung. Die UV-Strahlung weist hierbei Wellenlängen im Spektrum zwischen 100 nm und ca. 380 nm auf, die VIS-Strahlung weist hierbei Wellenlängen im Spektrum zwischen ca. 380 nm und ca. 780 nm auf, die nIR-Strahlung weist hierbei Wellenlängen im Spektrum zwischen ca. 780 nm und 1400 nm auf. Die genannten Spektren gehen ineinander über. Je nach medizinisch-kosmetischer Anwendung kann die Bestrahlung auf ein Teilspektrum der genannten Spektren konzentriert sein. Hierzu können medizinisch-kosmetische Strahlung emittierende Anordnungen auch dediziert einzelnen Wellenlängen zugeordnet sein, z.B. der UV-Strahlung, die von Strahlröhren erzeugt wird. Jedoch ist der Einsatz einer Strahlröhre nicht zwingend. Wegen der therapeutischen Effekte der medizinisch-kosmetischen Strahlung kann sie auch als medizinisch-kosmetisch-therapeutische Strahlung bezeichnet werden. Aus der Praxis sind Vorrichtungen zur Einwirkung auf die Haut eines Benutzers bekannt, wie sie beispielsweise in Sonnenstudios eingesetzt werden, bei denen eine zu bestrahlende Person zwecks Bräunung ihrer Haut durch Pigmentierung auf einer eine Liegefläche bzw. Abschlussfläche bildenden Abdeckung liegen kann, wobei unterhalb der Abdeckung eine UV-Strahlung emittierende Anordnung mit in der Regel einer Mehrzahl von Strahlröhren, insbesondere Leuchtstoffröhren, angeordnet ist, wobei die Abdeckung für den Zugang zu den Strahlröhren abgenommen bzw. verschwenkt werden kann. Meist weisen solche Bräunungsgeräte auch eine weitere Baueinheit mit weiteren Strahlröhren und einer zweiten Abdeckung auf, die gemeinsam auf die zu bestrahlende Person geschwenkt werden können, sodass die Person von zwei Seiten gebräunt werden kann. Problematisch ist hierbei, dass der Benutzer während der Bestrahlung die Betriebsparameter der Vorrichtung nicht ohne Weiteres einstellen kann, ohne seine Augen zu gefährden oder den Betrieb unterbrechen zu müssen.

Aus der Praxis sind auch sogenannte Stehbräuner bekannt, bei denen die zu bestrahlende Person nicht horizontal auf der Abdeckung liegt, sondern in vertikaler Position von der UV-Strahlung emittierenden Anordnung umgeben ist. Bei einem Stehbräuner verlaufen die Strahlrohre insbesondere in vertikaler Richtung.

US 2009/031491 A1 offenbart eine Vorrichtung gemäß dem Oberbegriff des unabhängigen Anspruchs 1.

Es ist die Aufgabe der Erfindung, eine Vorrichtung zum Einwirken auf zumindest Teile eines Körpers eines Benutzers anzugeben, mit der ein Benutzer insbesondere auch im laufenden Betrieb die Vorrichtung auf seine Bedürfnisse einstellen kann. Diese Aufgabe wird erfindungsgemäß durch eine Vorrichtung mit den Merkmalen eines unabhängigen Anspruchs gelöst.

Gemäß einem Aspekt der Erfindung ist eine Vorrichtung zum Einwirken auf zumindest Teile eines Körpers eines Benutzers, insbesondere mit medizinisch-kosmetischer Strahlung und/oder durch mechanische Beeinflussung, geschaffen, umfassend Mittel zum Einwirken auf den menschlichen Körper, die einstellbare Betriebsparameter umfassen, und eine mit den Mitteln zum Einwirken verbundene Steuerung, die die Betriebsparameter einstellt, die sich dadurch auszeichnet, dass zumindest ein Mikrofon vorgesehen ist, durch das Spracheingaben des Benutzers zur Steuerung der Betriebsparameter erfassbar sind, und dass das Mikrofon mit einer Auswerteeinheit verbunden ist, die von dem Mikrofon erfasste Spracheingaben in Steuerbefehle für die Mittel zum Einwirken umwandelt. Hierdurch ist vorteilhaft eine Möglichkeit geschaffen, mit geschlossenen Augen eine oder mehrere Betriebsparameter der Vorrichtung gemäß den Wünschen des Benutzers einzustellen, ohne dass der Benutzer hierfür seine Augen benutzen muss, was insbesondere auch bei sehbehinderten Personen vorteilhaft ist. Als weiterer Vorteil können die Betriebsparameter im laufenden Betrieb eingestellt werden, sodass die Behandlung, die mit dem Einwirken auf die Teile des Körpers einhergeht, nicht unterbrochen werden muss, sondern dynamisch an die Bedürfnisse des Benutzers anpassbar ist. Das Mikrofon stellt eine Schnittstelle für die Spracheingabe bereit, durch die der Benutzer Spracheingaben, insbesondere in Form von Befehlen, tätigen kann, ohne hierbei die Augen zu öffnen. Die von dem Mikrofon erfassten Spracheingaben werden in der Auswerteinheit konkreten Steuerbefehlen zugeordnet, die, wenn die Steuerbefehle eindeutig erkannt werden, an die Steuerung abgegeben werden, um die Mittel zum Einwirken entsprechend einzustellen.

Es versteht sich, dass Spracheingaben nicht nur während des Betriebs der Vorrichtung bzw. während der Dauer einer konkreten Anwendung möglich sind, sondern auch davor und/oder danach. So kann insbesondere auch die Effektbeleuchtung der Vorrichtung, in der Regel farbige LEDs im sichtbaren Lichtspektrum, eingestellt werden, beispielsweise durch Vorgabe der möglichen Parametergrößen Farbe oder Helligkeit.

Die Auswerteeinheit umfasst zweckmäßigerweise eine Rechnereinheit mit einer Spracherkennungseinrichtung, die die durch das Mikrofon erfassten Spracheingaben analysiert und die Steuerbefehle an die Steuerung übergibt.

Hierbei ist die Spracherkennungseinrichtung zweckmäßigerweise mit einer Anzahl von in einer Speichereinrichtung der Spracherkennungseinrichtung gespeicherten Begriffen ausgestattet, die bei Überschreiten eines voreinstellbaren Grades der Übereinstimmung der Spracheingabe als korrekte Eingabe gewertet werden. Durch das Vorsehen einer lokalen Spracherkennungseinrichtung in der Vorrichtung ist es nicht erforderlich, die Spracheingaben auf einen externen Server zu analysieren, und eine schnelle Reaktion auf die Spracheingabe ist möglich. Ferner kann durch die Vorgabe einer Menge von Begriffen, die in der Spracherkennungseinrichtung hinterlegt sind, eine große Fehlertoleranz bereitgestellt werden.

Erfindungsgemäß ist ein Lautsprecher in der Vorrichtung vorgesehen, wobei die Auswerteeinheit erfolgreich erkannte Spracheingaben mit einem akustischen Signal bestätigt. Das akustische Signal kann gemäß einer ersten Ausführungsvariante in der sprachlichen Wiedergabe des erkannten Steuerbefehls bestehen, wodurch der Benutzer in Falle einer Fehleingabe diese sofort erkennt und mittels neuerlicher Eingabe dem entgegenwirken kann. Es ist auch möglich, mittels einer entsprechenden Ansage Fragen an den Benutzer zu prompten, die dieser mit ja oder mit nein oder mit anderen eindeutigen Angaben beantworten kann, wenn die Spracheingabe keinem Steuerbefehl eindeutig zugeordnet werden konnte, oder wenn die Übereinstimmung mit den gespeicherten Sprachdaten einen voreinstellbaren Schwellenwert unterschreitet. Besonders bevorzugt ist jedoch das Quittieren der erfolgreichen Erkennung der Spracheingabe durch ein akustisches Signal, das dem Benutzer vermittelt, dass die Spracheingabe und der damit verbundene Befehl erkannt wurde. Das akustische Signal kann auch mit einem taktilen Signal kombiniert sein, z.B. einem Vibrieren.

Die Auswerteeinheit ist zweckmäßigerweise durch Spracheingabe eines Schlüsselwortes aktivierbar. Das Aktivieren der Auswerteeinheit bewirkt, dass die nachfolgenden erfassten Spracheingaben mit in der Auswerteeinheit gespeicherten Begriffen verglichen werden, um festzustellen, ob es sich um zulässige Steuerbefehle handelt. Hierdurch wird verhindert, dass auf Grund von Unterhaltungen, Geräuschen von externen Quellen, abgespielten Rundfunkbeiträgen oder zufällig benutzten Begriffen im Zuge eines Gesprächs die Betriebsparameter der Mittel zum Einwirken auf den menschlichen Körper zufällig verändert werden. Die Erkennung des Schlüsselworts stellt hierbei sicher, dass der Benutzer bewusst eine Spracheingabe vornehmen möchte, und verhindert überdies, dass zufällig mit Steuerbefehlen übereinstimmende verwendete Begriffe zu fälschlichen Veränderungen der Betriebsparameter führen.

In weiterer vorteilhafter Ausgestaltung ist vorgesehen, dass die Auswerteeinheit durch Berühren eines berührungsempfindlichen Bildschirms aktivierbar ist. Damit ist eine taktile Interaktion zum Aktivieren der Auswerteeinheit möglich, die es dem Benutzer auch dann ermöglicht, Spracheingaben vorzunehmen, wenn das Schlüsselwort nicht bekannt ist oder von der Auswerteeinheit nicht erkannt wird. Auch das Berühren des Bildschirms kann mit einem akustischen Signal quittiert werden, um die Bereitschaft zur Entgegennahme von Spracheingaben dem Benutzer zu signalisieren.

Gemäß noch einer weiteren bevorzugten Ausgestaltung ist vorgesehen, dass eine gesonderte Taste oder ein Druckknopf vorgesehen ist, wobei durch Betätigen der Taste die Auswerteeinheit aktivierbar ist. Die Taste ist zweckmäßigerweise neben dem Mikrofon angeordnet, wodurch sprachliche Eingaben eines Benutzers im Unterbewusstsein in Richtung auf das Mikrofon gerichtet werden. Es ist alternativ auch möglich, die entsprechende Taste im Bereich einer Handauflagefläche für den Benutzer zu positionieren. Statt einer Taste kann auch eine berührungsempfindliche Folie oder ein elektrischer Kontakt vorgesehen sein, der durch manuelle Berührung aktivierbar ist.

Vorzugsweise weist die Vorrichtung zumindest ein Display und zumindest einen Lautsprecher auf, wodurch über das Display und den Lautsprecher optische und/oder akustische Erläuterungen der Sprachbefehle anzeigbar sind. So können beispielsweise zu einem Zeitpunkt, zu dem der Benutzer seine Augen noch einsetzen kann, also vor der entsprechenden Behandlung, über ein Erklärvideo die möglichen Spracheingaben für Steuerbefehle und die möglichen Parametergrößen für die Spracheingaben schriftlich und akustisch erläutert werden. Dies ist insbesondere dann vorteilhaft, wenn fremdsprachliche Benutzer die Vorrichtung mittels Spracheingabe steuern möchten, die mit der Aussprache der verwendeten Begriffe nicht vertraut sind. Zugleich kann hierdurch vorteilhaft die Funktionsweise der Vorrichtung erklärt werden und die einstellbaren Parameter illustriert werden. Ein besonderer Vorteil besteht darin, dass bei sukzessivem Ausbau der Spracheingabe entsprechende Informationen aktualisiert werden können. Zugleich können über das Display auch Werbefilme, Informationen des Betreibers der Vorrichtung und dergleichen ausgestrahlt werden.

In besonders zweckmäßiger Weiterbildung ist das Mikrofon der Vorrichtung mit einer zu der Vorrichtung externen Leitstelle verbunden und ermöglicht damit Rückfragen bezüglich des Wohlbefindens des Benutzers. So kann beispielsweise dann, wenn die Behandlung lange dauert, eine Bedienperson der Leitstelle Kontakt mit dem Benutzer aufnehmen und sich durch dessen Antwort vergewissern, dass sowohl die Vorrichtung als auch der Benutzer keinen Beeinträchtigungen unterliegen.

In entsprechender Weise kann das Mikrofon der Vorrichtung mit einem zu der Vorrichtung externen Anbieter verbunden werden, beispielsweise über das Internet. So kann der Benutzer das Mikrofon auch ohne Steuerbefehle zu erzeugen nutzen, um beispielsweise mit einer anderen Person zu telefonieren oder um auf Inhalte im Internet zuzugreifen. Außerhalb der Behandlung können überdies über die in der Vorrichtung vorgesehenen Displays auch optische Inhalte aus dem Internet wiedergegeben werden. Hierdurch ergibt sich ein weiterer oder zusätzlicher Nutzen für den Benutzer, der das Mikrofon auch für die Kommunikation mit außenstehenden Anbietern nutzen kann.

Gemäß einer besonders günstigen Verwirklichung ist vorgesehen, dass bei Erfassung einer Spracheingabe, insbesondere bei Erfassung des Schlüsselwortes oder bei Aktivierung der Auswerteeinheit auf andere Weise die mit einer Lautstärkeentwicklung verbundenen Betriebsparameter durch die Steuerung abgesenkt bzw. reduziert werden. Hierbei können nicht nur Betriebsparameter der Mittel zum Einwirken auf den menschlichen Körper abgesenkt werden, sondern auch andere Betriebsparameter, die insbesondere mit einer Geräuschentwicklung verbunden sind, beispielsweise die Wiedergabe von Musik und dergleichen. Besonders bevorzugt werden bei Erfassung einer Spracheingabe die Betriebsparameter reduziert, die Geräusche entwickeln, wie beispielsweise die Lüftung der Vorrichtung, indem die Drehzahl des Gebläses reduziert wird, oder aber die Lautstärke von wiedergegebener Musik, indem die Musik entweder ganz abgeschaltet wird oder deren Lautstärke reduziert wird.

Erfindungsgemäß ist vorgesehen, dass über Lautsprecher der Vorrichtung wiedergegebene akustische Signale über einen separaten Eingang der Auswerteeinheit zugeführt werden, und dass diese invertiert werden, damit die zufällig durch das Mikrofon erfassten Geräusche ausdrücklich nicht als Steuerbefehl verstanden werden. So ist es beispielsweise möglich, bei Abspielen eines Wortbeitrages, wie beispielsweise eines Beitrags eines Radiomoderators, diesen in der Auswerteeinheit zu neutralisieren, selbst dann, wenn der Beitrag das Schlüsselwort verwendet. Hierdurch kann in besonders günstiger Weise sichergestellt werden, dass eine zufällige Aktivierung der Auswerteeinheit, die das Erlebnis des Einwirkens beeinträchtigen könnte, zuverlässig verhindert ist. Vorzugsweise wird eine voreinstellbare Zeit nach Ende einer Eingabe die Absenkung des Betriebsparameters rückgängig gemacht, wodurch dem Benutzer signalisiert wird, dass die Auswerteeinheit wieder deaktiviert ist.

Zweckmäßigerweise ist für die Spracheingabe der Betriebsparameter zu bezeichnen, der eingestellt werden soll, wobei der Betriebsparameter aus einer Menge von Betriebsparametern, die in der Auswerteeinheit gespeichert sind, ausgewählt ist. Der entsprechende Betriebsparameter kann durch eine Parametergröße qualifiziert werden, sodass die Kombination von Betriebsparameter und Parametergröße eine einfache und wirksame Einstellung der Mittel zum Einwirken auf den Körper ermöglicht. Darüber hinaus können auch Betriebsparameter, die nicht die Mittel zum Einwirken auf den Körper betreffen, eingestellt werden, zum Beispiel die Beleuchtung oder die Temperatur in dem Raum, in dem die Vorrichtung aufgestellt ist, oder die Lautstärke der Musikwiedergabe.

Die Betriebsparameter sind zweckmäßigerweise ausgewählt aus der Gruppe umfassend Gesichtslampen, Körperlampen, LEDs, Körperlüftung, Gesichtslüftung, Temperatur, Feuchtigkeitszugabe, Musikwiedergabe, Lautstärke, Aromazugabe, Rotlicht, Blaulicht, Wasserstrahlstärke, Massageintensität, Massageprogramm, Lichtprogramm. Es ist hierbei nicht erforderlich, dass der jeweilige Betriebsparameter die genaue technische Funktion bezeichnet. So wird zur Einstellung der Intensität der Körperlüftung die Drehzahl einer entsprechenden Pumpe eingestellt, ohne dass die Pumpe hierfür konkret bezeichnet werden muss.

Zusätzlich zu dem Betriebsparameter kann eine Menge von Parametergrößen vorgegeben sein, die den Betriebsparameter charakterisieren. Hierbei ist die Parametergröße zweckmäßigerweise ausgewählt aus der Gruppe umfassend Plus, Minus, Maximum, Minimum, Standard, Ein, Aus. Die Parametergröße "Ein" bzw. "Aus" bezeichnet, dass der konkrete Betriebsparameter angeschaltet bzw. ausgeschaltet werden soll. Beispielsweise kann Licht oder Musik auf die Weise angeschaltet oder ausgeschaltet werden. Es ist aber auch möglich, die Behandlung insgesamt ein- oder auszuschalten. Die Parametergröße "Plus" und "Minus" bezeichnet, dass bei skalierbaren Betriebsparametern der entsprechende Betriebsparameter inkrementiert bzw. dekrementiert werden soll. Auf diese Weise ist es vorteilhaft möglich, in mehreren Stufen unterteilte Betriebsparameter zu erhöhen oder abzusenken, ohne dass hierfür die konkrete Zahl ausgewählt werden oder bekannt sein muss. Hierdurch wird vorteilhaft ermöglicht, dass der Benutzer situativ als zu stark oder zu schwach ausgebildete Betriebsparameter anpasst, ohne hierfür den absoluten Wert des Betriebsparameters kennen zu müssen. Hierdurch wird insbesondere vorteilhaft erreicht, dass der Benutzer den Betriebsparameter individuell an seine Bedürfnisse während der Behandlung anpassen kann. Die Parametergrößen "Maximum", "Minimum" und "Standard" ermöglichen es dem Benutzer, einen Betriebsparameter auf die maximale, minimale oder standardisierte, in der Regel mittlere Stufe einzustellen, ohne hierfür wissen zu müssen, auf welcher Stufe sich der Betriebsparameter aktuell befindet. Hierdurch ist es für den Benutzer einfach möglich, ausgehend von einer konkreten Einstellung des Betriebsparameters die Behandlung vollziehen zu lassen.

Es ist ferner möglich, mit der Spracheingabe konkrete Bezeichnungen der Mittel zum Einwirken vorzugeben, mittels derer eine Behandlung in die Wege geleitet wird. So kann die Spracheingabe "Massage Ein" angeben, dass eine Massageanordnung zum Einwirken auf den Körper eingeschaltet werden soll. So kann die Spracheingabe "Körperlüftung Plus" angeben, dass eine Körperbelüftungsanordnung zum Einwirken auf den Körper um eine Stufe erhöht werden soll. So kann die Spracheingabe "Gesichtslampen Maximum" angeben, dass eine Gesichtslampen umfassende Anordnung zum Einwirken auf den Körper, vorliegend auf das Gesicht des Benutzers mittels medizinisch-kosmetischer Strahlung auf die maximale Leistung eingestellt werden soll.

Es ist möglich, weitere Begriffe zu definieren, die von der Auswerteeinheit als Befehle verstanden werden, die nicht in Steuerbefehle umgewandelt werden. Bewirkt der Benutzer beispielweise die Spracheingabe "Telefon", kann die Auswerteeinheit veranlassen, dass ein mit der Vorrichtung gekoppeltes oder koppelbares Mobiltelefon des Benutzers zum Telefonieren bereitgestellt wird. Die Spracheingabe "Internet" kann beispielweise bewirken, dass der Benutzer auf das Internet zugreifen kann, insbesondere auf die Spracheingabe seines Mobilgeräts, zum Beispiel seiner Suchmaschine.

Die Teile des Körpers, auf die die Vorrichtung einwirkt, sind zweckmäßigerweise ausgewählt aus der Gruppe umfassend die Haut, das Bindegewebe, die Muskulatur und die Knochen. Insbesondere bei der Einwirkung auf den Körper bzw. seine Teile mittels mechanischer Beeinflussung wird das Bindegewebe und die Muskulatur regeneriert, beispielsweise durch elastische Deformation der entsprechenden Körperteile. Hierdurch wird die Durchblutung der Körperteile angeregt und verbessert und eine Regenerationswirkung erzielt. Die Übertragung der entsprechenden Kräfte auf den Körper bzw. seine Teile kann auf vielfältige Weise erfolgen, beispielsweise durch pneumatische oder hydraulische Wasserdüsen, die unmittelbar auf den Körper einwirken oder mittelbar über eine membranartige Zwischenlage. Die membranartige Zwischenlage kann hierbei eine Liegefläche ausbilden, auf der der Benutzer sich legen kann. Andere Mittel zum Einwirken auf den menschlichen Körper nutzen Strahlung unterschiedlicher Wellenlänge, insbesondere ultraviolette Strahlung, sichtbare Strahlung und infrarote Strahlung. Die medizinisch-kosmetische Strahlung wirkt auf die äußere Hautschicht, aber auch auf darunter liegende Bereiche des Körpers und regt insbesondere die Pigmentierung der Haut an. Darüber hinaus werden in Abhängigkeit von der Wellenlänge und dem betroffenen Körperteil unterschiedlich tiefe Partien des Körpers erreicht und damit auf diese eingewirkt.

In einer besonders günstigen Ausgestaltung ist vorgesehen, dass spezifische Sprachparameter des Benutzers auf einer Speichereinheit gespeichert sind, beispielsweise auf einer für den Benutzer spezifischen Kundenkarte. Diese spezifischen Sprachparameter können nach Einlesen der Daten über eine hierzu bereitgestellte Schnittstelle von der Auswerteeinheit verwertet werden, um spezifische Aussprachen des Benutzers bei der Auswertung der erfassten Spracheingaben zu berücksichtigen. Insbesondere kann dort auch ein individuelles Schlüsselwort gespeichert sein, das der Benutzer verwenden möchte. Es ist möglich, weitere Informationen, die den Benutzer betreffen, auf der Speichereinheit zu speichern, diese ermöglichen, den Benutzer mit Namen zu begrüßen oder seine Basiseinstellung für die Vorrichtung einzustellen.

Zweckmäßigerweise stellt die Steuerung auch andere als die Mittel zur Einwirkung auf den menschlichen Körper ein, beispielsweise die Lautstärke von Musik oder die Umgebungstemperatur oder die Beleuchtung, jeweils außerhalb der Vorrichtung.

Gemäß einer günstigen Ausgestaltung ist vorgesehen, dass die Vorrichtung als Hydromassage-Vorrichtung mit einer membranartigen Liegefläche für den Benutzer ausgebildet ist, die zumindest eine unterhalb der Liegefläche aktivierbare Massagedüse ausweist, die einen Massagewasserstrahl gegen die membranartige Liegefläche zum Massieren des darauf liegenden Benutzers richten kann. Es versteht sich, dass gleichzeitig mehrere Massagedüsen in Abstimmung aufeinander für ein symmetrisches Massageerlebnis oder unabhängig voneinander zur gleichzeitigen Bearbeitung unterschiedlicher Körperregionen vorgesehen sein können.

Gemäß einer anderen Ausgestaltung ist vorgesehen, dass die Mittel zum Einwirken ausgewählt sind aus der Gruppe umfassend UV-Licht emittierende Leuchtstoffröhren, sichtbares Licht emittierende Leuchtstoffröhren, UV-Licht emittierende LEDs, sichtbares Licht emittierende LEDs, UV-Licht emittierende Hochdrucklampen. Die vorgenannten Mittel sind jeweils dazu ausgebildet, ein vorbestimmtes Teilspektrum von medizinisch-kosmetischer Strahlung auszusenden, die beispielsweise eine Gesichtsbräunung oder eine Bräunung des Körpers erreicht. Hierbei können die unterschiedlichen Mittel individuell oder in Gruppen eingestellt werden.

Nach einer bevorzugten Weiterbildung ist vorgesehen, dass das Mikrofon als Mikrofon eines tragbaren Mobilgeräts ausgebildet ist, das an die Vorrichtung drahtgebunden oder drahtlos anschließbar ist, wodurch der Einbau des Mikrofons in dem Gehäuse der Vorrichtung überflüssig ist. Durch die Verbindung mit der Auswerteeinheit kann das externe Mikrofon Spracheingaben erfassen. Es ist also möglich, dass die Auswerteeinheit auch auf dem tragbaren Mobilgerät implementiert ist, beispielsweise in einer Software-Applikation für das Mobilgerät, wodurch dann nur noch die Steuerbefehle von dem Mobilgerät an die Steuereinheit abgeliefert werden müssen. Bevorzugt ist allerdings das Mikrofon in einem Gehäuse der Vorrichtung eingebaut.

Gemäß einem Aspekt der Erfindung ist eine Vorrichtung zum Einwirken auf zumindest Teile eines Körpers eines Benutzers mit medizinisch-kosmetischer Strahlung geschaffen, umfassend ein selbsttragendes Gehäuse, in dem eine medizinisch-kosmetische Strahlung emittierende Anordnung zum Einwirken auf den menschlichen Körper mit einstellbaren Betriebsparametern untergebracht ist, und eine mit der Anordnung zum Einwirken verbundene Steuerung, die die Betriebsparameter einstellt, wobei sich die Vorrichtung dadurch auszeichnet, dass an dem Gehäuse zumindest ein Mikrofon vorgesehen ist, durch das Spracheingaben des Benutzers zur Steuerung der Betriebsparameter erfassbar sind, und dass das Mikrofon mit einer Auswerteeinheit verbunden ist, die von dem Mikrofon erfasste Spracheingabe in Steuerbefehle für die Anordnung zum Einwirken umwandelt. Hierdurch ist vorteilhaft eine insbesondere als Bräunungsvorrichtung ausgebildete Vorrichtung geschaffen, die durch Spracheingaben gesteuert werden kann. Insbesondere können die Bestandteile der medizinisch-kosmetischen Strahlung emittierenden Anordnung durch verbale Eingabe von Steuerbefehlen eingestellt werden, ohne dass der Benutzer hierfür von seinen Augen Gebrauch machen muss, mit der vorteilhaften Wirkung, dass die möglicherweise für das Augenlicht schädliche Einwirkung medizinisch-kosmetischen Strahlung auf die Sehorgane vermieden wird. Darüber hinaus kann sich der Benutzer auf die verbleibenden Sinne konzentrieren, was zur entspannenden Wirkung beiträgt. Der Benutzer ist nicht mehr gezwungen, für eine Einstellung der Betriebsparamater die Behandlung zu unterbrechen, wodurch der Nutzungsgrad der Vorrichtung erhöht wird.

Zweckmäßigerweise sind die Mittel zum Einwirken in einem selbsttragenden Gehäuse untergebracht. Dadurch ist vorteilhaft sichergestellt, dass die Mittel zum Einwirken zuverlässig gekapselt sind und die Mittel zum Einwirken gegen Staub und Umgebungseinflüsse geschützt sind.

Vorzugsweise umfasst das Gehäuse ein stationäres Gehäuseteil und ein bewegliches Gehäuseteil, wobei das bewegliche Gehäuseteil über eine Anlenkachse mit dem stationären Gehäuseteil gekoppelt ist. Das bewegliche Gehäuseteil und das stationäre Gehäuseteil bilden hierbei zweckmäßig eine vertikale oder eine horizontale Röhre aus, die jedoch keinen zylindrischen Querschnitt ausweisen muss. Die Gehäuseteile sind vorzugsweise als für medizinisch-kosmetische Strahlung zumindest überwiegend transparentes Polymethylmethacrylat, das auch als Acrylglas bezeichnet wird, ausgebildet, durch das die Strahlung mit geringen Verlusten durchtreten kann.

Zweckmäßigerweise umfasst das Gehäuse ein stationäres Gehäuseteil und ein bewegliches Gehäuseteil, wobei das bewegliche Gehäuseteil über eine Anlenkachse mit dem stationären Gehäuseteil gekoppelt ist. Hierdurch wird vorteilhaft erreicht, dass das Gehäuse für den Zugang des Benutzers aufgeschwenkt und vor der Einwirkung auf den Benutzer geschlossen werden kann.

In vorteilhafter Weiterbildung ist vorgesehen, dass das stationäre Gehäuseteil eine Liegefläche für den Benutzer ausbildet, die überwiegend eben ist. Das bewegliche Gehäuseteil ist dann zur Bildung einer Röhre auf das stationäre Gehäuseteil herabschwenkbar. Hierdurch ist vorteilhaft die Möglichkeit geschaffen, dass der Benutzer liegend in der Vorrichtung, nämlich mit dem Rücken auf der Liegefläche, Platz nehmen kann.

Zweckmäßigerweise ist ein Mikrofon an dem beweglichen Gehäuseteil angeordnet, sodass der mit dem Rücken auf der Liegenfläche liegende Benutzer mit seinem Gesicht dem Mikrofon zugekehrt ist. Hierdurch kann das Mikrofon von dem Benutzer ausgehenden Schallwellen gut erfassen.

Gemäß einer bevorzugten Ausgestaltung ist das Mikrofon an einem axialen Ende des beweglichen Gehäuseteils angeordnet. Hierdurch ist insbesondere vorteilhaft sichergestellt, dass die Drähte, die für die Kontaktierung des Mikrofons erforderlich sind, nicht durch das Acrylglas von dem Benutzer gesehen werden können. Darüber hinaus ist das Mikrofon damit außerhalb der wesentlichen Bereiche der Strahlung angeordnet, und damit ist die Gefahr, dass die metallischen Komponenten des Mikrofons aufgeheizt werden, reduziert. Zweckmäßigerweise ist das Mikrofon an einem axialen Kopfende des beweglichen Gehäuseteils angeordnet.

Gemäß einer bevorzugten Ausgestaltung ist vorgesehen, dass das Mikrofon in einer Bohrung einer das Gehäuseteil ausbildenden, für medizinisch-kosmetische Strahlung zumindest überwiegend transparenten stabilen Kunststoffhülle angeordnet ist. Die Bohrung stellt sicher, dass die von dem Benutzer ausgehenden Schallwellen zuverlässig erfasst werden.

Zweckmäßigerweise ist das Mikrofon außerhalb des Bereichs der unmittelbaren Einwirkung der medizinisch-kosmetischen Strahlung angeordnet, sodass weder Alterungseffekte auf Grund des UV-Lichts noch temperaturinduzierte Effekte am Mikrofon auftreten.

Zweckmäßigerweise ist das Mikrofon im Bereich eines das Gehäuseteil, insbesondere das bewegliche Gehäuseteil, aussteifenden Rahmenabschnitts des Gehäuses angeordnet, wodurch sich der Anschluss des Mikrofons optisch günstig kaschieren lässt.

Gemäß einem Aspekt der Erfindung ist ein Verfahren zum Einwirken auf zumindest Teile eines Körpers eines Benutzers, insbesondere mit kosmetisch-medizinischer Strahlung und/oder durch mechanische Beeinflussung, umfassend Mittel zum Einwirken auf den menschlichen Körper, die einstellbare Betriebsparameter umfassen, und eine mit den Mitteln zum Einwirken verbundene Steuerung, die die Betriebsparameter einstellt, bei dem der Benutzer durch Spracheingabe zumindest einige der Betriebsparameter vorgibt, und bei dem die von mindestens einem Mikrofon erfassten Spracheingaben durch eine Auswerteeinheit in Steuerbefehle für die Mittel zum Einwirken umgewandelt werden. Hierdurch wird vorteilhaft erreicht, dass Benutzer, die Ihre Augen nicht einsetzen können oder dürfen - in Vorrichtungen zum Einwirken auf die Haut mittels UV-Strahlung ist das die Regel - gleichwohl im laufenden Betrieb Änderungen von Einstellungen der Vorrichtung zum Einwirken, insbesondere der Mittel zum Einwirken vornehmen können, wodurch sich das Wohlbefinden der Benutzer und deren Treue gegenüber dem Produkt erhöht.

Das Verfahren kann insbesondere auch als Verfahren zum Betreiben der Vorrichtung, insbesondere der vorstehend dargelegten Vorrichtung, genutzt werden, so dass sich ein Verfahren zum Betreiben einer Vorrichtung zum Einwirken ergibt, das die Merkmale des vorgenannten Verfahrens aufweist.

Vorzugsweise wird die Auswerteeinheit durch Spracheingabe eines Schlüsselwortes aktiviert, und die der Spracheingabe des Schlüsselwortes folgenden Spracheingaben werden in der Auswerteeinheit auf Übereinstimmung mit gespeicherten Befehlen für Betriebsparameter verglichen. Hierdurch wird vorteilhaft erreicht, dass versehentliche Benennungen einzelner Steuerbefehle nur dann beachtet werden, wenn die Auswerteeinheit durch die Nennung des Schlüsselwortes zuvor in einen Zustand der aktiven Erfassung der Spracheingaben versetzt wurde. Hierdurch werden unbeabsichtigte Fehlbedienungen vermieden.

In günstiger Weiterbildung ist vorgesehen, dass die in der Auswerteeinheit gespeicherten Betriebsparameter ausgewählt sind aus der Gruppe umfassend Gesichtslampen, Körperlampen, LEDs, Körperlüftung, Gesichtslüftung, Temperatur, Feuchtigkeitszugabe, Musikwiedergabe, Lautstärke, Aromazugabe, Rotlicht, Blaulicht, Wasserstrahlstärke, Massageintensität, Massageprogramm, Lichtprogramm.

Die Betriebsparameter werden zweckmäßigerweise durch Parameter qualifiziert, die ebenfalls mittels Spracheingabe erfassbar sind. Vorzugsweise sind in der Auswerteeinheit zu den gespeicherten Betriebsparametern Parametergrößen gespeichert, die ausgewählt ist aus der Gruppe umfassend Plus, Minus, Maximum, Minimum, Standard, Ein, Aus.

Eine verbesserte Identifizierung der Spracheingabe ergibt sich, wenn bei Erfassen einer Spracheingabe mit einer Lautstärkeentwicklung verbundene Betriebsparameter durch die Steuerung abgesenkt werden, und dass eine voreinstellbare Zeit nach Ende einer Eingabe die Absenkung rückgängig gemacht wird. Hierzu kann vorteilhaft die Spracheingabe eines Schlüsselwortes, zum Beispiel eines Vornamens oder eines Namens, beispielsweise Eva, Aphrodite, Hera oder Venus, zum Aktivieren der Auswerteeinheit vorgesehen werden.

Es versteht sich, dass das Verfahren auch durch die vorstehend im Zusammenhang mit den Vorrichtungen aufgeführten Merkmale weitergebildet werden kann. Insbesondere ist das Verfahren auf einer entsprechend ausgebildeten Vorrichtung ausführbar.

Weitere Vorteile, Merkmale, Eigenschaften und Weiterbildungen der Erfindung ergeben sich aus der nachfolgenden Beschreibung eines bevorzugten Ausführungsbeispiels sowie aus den abhängigen Ansprüchen.

Die Erfindung wird nachstehend unter Bezugnahme auf die anliegenden Zeichnungen anhand eines bevorzugten Ausführungsbeispiels näher erläutert.
- Fig. 1: zeigt eine schematische Seitenansicht auf ein bevorzugtes Ausführungsbeispiel einer erfindungsgemäßen Vorrichtung.

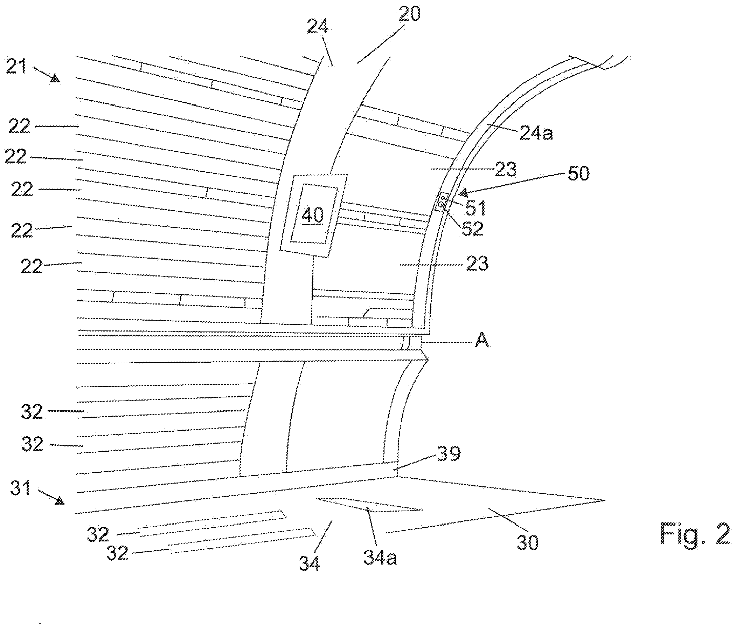

- Fig. 2: zeigt eine perspektivische Ansicht der Vorrichtung aus Fig. 1

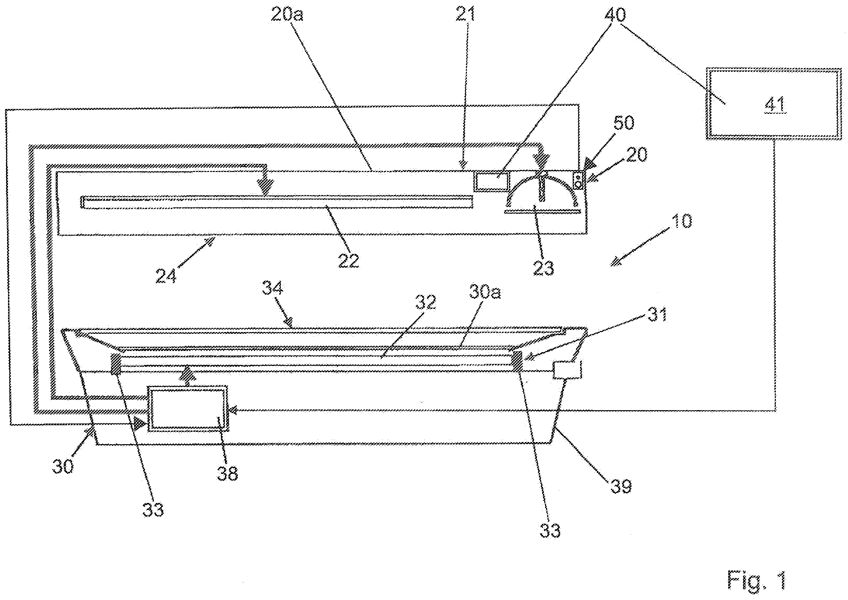

In Fig. 1 ist eine als Bräunungseinrichtung ausgebildete Hautbestrahlungsvorrichtung 10 dargestellt, die mittels medizinisch-kosmetischer Strahlung auf zumindest die Haut und zum Teil die darunter liegenden Partien des Körpers einwirkt. Die Vorrichtung 10 umfasst ein bewegliches Oberteil 20 und ein stationäres Unterteil 30. Das Oberteil 20 kann über ein nicht im Einzelnen dargestelltes Scharnier entlang einer Anlenkachse A nach unten geschwenkt werden, sodass ein vergleichsweise kleiner Raum in Gestalt einer Röhre für eine liegende zu bestrahlende Person, den Benutzer, geschaffen ist.

Das Oberteil 20 umfasst eine medizinisch-kosmetische Strahlung emittierende Anordnung 21 mit einer Mehrzahl von UV-Strahlung emittierenden Strahlröhren 22, die in einer horizontalen Richtung eingebaut sind, sowie einer als Gesichtsbräuner ausgebildeten weiteren Strahlvorrichtung 23 auf der Basis von UV-Strahlung emittierenden LEDs. Das Oberteil 20 ist in einem Gehäuseteil 24 angeordnet, wobei das Gehäuse 24 zumindest abschnittsweise aus für UV-Stahlung transparentem Polymethylmethacrylat bzw. Acrylglas gebildet ist. Die Wandstärke des Polymethylmethacrylats ist recht dünn bemessen, da es keine signifikanten mechanischen Lasten aufnehmen muss. In dem Oberteil 20 ist ferner ein Touch-Display 40 untergebracht, wobei das Touch-Display mit seiner Bedienoberfläche 41 dem Unterteil 30 zugekehrt ist. Es ist möglich, einen äußeren Deckel 20a des Oberteils 20 zu öffnen, um Zugang zu den Leuchtstoffröhren 22 zu bekommen.

Das Unterteil 30 umfasst ein Gehäuseteil 39, das nach Art eines Sockels ausgebildet ist, und in dem eine Steuerung 38, die elektrische Versorgung sowie weitere Teile der Hautbestrahlungseinrichtung 10 wie Lüfter, Schütze, usw. untergebracht sind. Das Gehäuse 39 umfasst weiterhin eine medizinisch-kosmetische Strahlung emittierende Anordnung 31, mit einer Mehrzahl von UV-Strahlung emittierenden Leuchtstoffröhren 32 sowie mit jeder der Leuchtstoffröhren 32 endseitig verbundene elektronische Vorschaltgeräte 33.

Oberhalb der UV-Strahlen emittierenden Anordnung 31 und oberhalb der Strahlröhren 32 ist ein Deckel 30a aus Polymethylmethacrylat vorgesehen, der die Anordnung 31 bedeckt. Oberhalb des Deckels 30a ist eine Abdeckung 34 vorgesehen, die eine dem Oberteil 20 zugekehrte obere Abschlussfläche aufweist. Eine Nackenstütze 34a auf der Abschlussfläche 34 zentriert den Kopf des Benutzers, so dass die Gesichtsbräuner 23 dem Gesicht des Benutzers zugekehrt sind, und die Strahlröhren 22, 32 dem Rumpf des Benutzers. Hierdurch ist auch der Mund des Benutzers recht genau in seiner Position definiert.

Legt sich nun eine zu bestrahlende Person auf die Abschlussfläche 34, kann diese, beispielsweise ausgehend von einem voreingestellten Standardwert, über die Steuerung 38 die Leistung der UV-Strahlung emittierenden Anordnungen 21, 31, 23 steuern, und zwar jeweils unabhängig voneinander. Möchte die zu bestrahlende Person nun die Leistung der UV-Strahlung emittierenden Anordnungen 21 und/oder 31 verändern, kann sie über ein Menü in dem Touch-Display 40, das mit der Steuerung 38 verbunden ist, entsprechende Befehle eingeben. Das Touch-Display 40 kann dann fest in der Hautbestrahlungseinrichtung 10 eingebaut werden, insbesondere im Bereich des dem Gesicht der zu bestrahlenden Person zugekehrten Oberteils 20. Es ist möglich, über in dem Oberteil 20 eingebaute Lautsprecher auch Musik oder dergleichen wiederzugeben.

Problematisch bei der Eingabe von Befehlen über das Touch-Display 40 ist jedoch der Umstand, dass dies im Prinzip vor der Einwirkung der UV-Strahlung auf den Körper des Benutzers erfolgen muss. Denn sobald die UV-Strahlung emittierenden Anordnungen 21, 31, 23 eingeschaltet sind, muss der Benutzer seine Augen geschlossen halten und je nach anwendbarer Vorschrift sogar eine Schutzbrille tragen. Durch die Schutzbrille lässt sich jedoch das Touch-Display 40 nicht zuverlässig ablesen. Daher ist in dem oberen Gehäuseteil 24 zusätzlich eine akustische Bedieneinheit 50 ausgebildet, die aus einem Mikrofon 51 und einer als Druckknopf ausgebildeten Taste 52 besteht.

Die akustische Bedieneinheit 50 ist hierbei an einem axialen Ende des beweglichen oberen Gehäuseteils 24 angeordnet, und zwar in etwa an dem höchsten Punkt der gebildeten Röhre, wenn das oberen Gehäuseteil 24 herabgeschwenkt ist. Zugleich ist die akustische Bedieneinheit 50 an einem proximalen Ende des oberen Gehäuseteils 24 angeordnet, in dem auch ein das Gehäuseteil 24 aussteifender Rahmenabschnitt 24a ausgebildet ist. Damit befindet sich das Mikrofon 51 außerhalb der unmittelbaren Strahlung der Strahlvorrichtung 23, so dass es nicht in schädlicher Weise aufgeheizt wird. Um die Schallwellen des Benutzers erfassen zu können, ist das Acrylglas-Material des oberen Gehäuseteils 24 von einer zylindrischen Bohrung durchsetzt, in der das Mikrofon 51 eingesetzt ist.

Die Steuerung 38 umfasst eine Auswerteeinheit mit einem Speicher, in dem Spracheingaben gespeichert sind, wobei die Auswerteeinheit mittels einer Software in der Lage ist, Spracheingaben der Benutzer auf Übereinstimmung mit gespeicherten Befehlen zu vergleichen. Ist ein Schwellenwert der Übereinstimmung mit einem gespeicherten Steuerbefehl überschritten, wird die Spracheingabe als dieses Steuerbefehl betreffend zugeordnet, und die Steuerung 38 veranlasst, dass ein entsprechender Steuerbefehl an die Mittel zum Einwirken 22, 32, 23 abgegeben wird. Neben den Mitteln zum Einwirken durch medizinisch-kosmetische Strahlung kommen auch weitere Mitteln zum Einwirken in Betracht, zum Beispiel eine zirkulierende Körperlüftung, eine Gesichtslüftung, der Feuchtigkeitsanteil in der Lüftung und weitere mehr. Darüber hinaus können auch weitere Betriebsparameter eingestellt werden, zum Beispiel die Lautstärke der Musik, das Musikprogramm und weitere mehr.

Die Auswerteeinheit wird wahlweise durch das Aussprechen eines Schlüsselwortes, z.B. einen Namen des Gerätes, beispielsweise "Eva", durch das Drücken der Taste 52 oder durch das Berühren des Displays 40 aktiviert, d.h. die nachfolgend ausgesprochenen Befehle werden auf Übereinstimmung mit zulässigen Steuerbefehlen verglichen. Solange die Auswerteeinheit für Spracheingaben empfangsbereit ist, werden Geräusche verursachende Betriebsparameter reduziert, zum Beispiel Musikwiedergabe, zirkulierende Körperlüftung und dergleichen.

Die Spracheingabe umfasst hierbei einen Betriebsparameter, wie zum Beispiel Gesichtslampen, Körperlampen, LEDs, Körperlüftung, Gesichtslüftung, Temperatur, Feuchtigkeitszugabe, Musikwiedergabe, Lautstärke, Aromazugabe, Rotlicht, Blaulicht, Wasserstrahlstärke, Massageintensität, Massageprogramm, Lichtprogramm. Zusätzlich zu dem Betriebsparameter ist eine Menge von Parametergrößen vorgegeben, die den Betriebsparameter charakterisieren. Die Parametergröße lautet zum Beispiel "Plus" für Inkrementieren des Betriebsparameters, "Minus" zum Dekrementieren des Betriebsparameters, "Maximum" zum Einstellen des maximalen Wertes des Betriebsparameters, "Minimum" zum Einstellen des minimalen Wertes des Betriebsparameters, "Standard" zum Einstellen eines mittleren Wertes des Betriebsparameters, "Ein" zum Einschalten des Betriebsparameters und "Aus" zum Ausschalten des Betriebsparameters.

Spricht der Benutzer etwa "Eva Gesichtslampen Maximum" aus, erkennt die Auswerteeinheit durch das Schlüsselwort "Eva", dass sie aktiviert wird und dass es sich bei dem nachfolgenden Spracheingaben um Befehle handelt. Werden die Worte "Gesichtslampen Maximum" dann richtig erkannt, veranlasst die Steuerung 38 die Gesichtslampen 23 dazu, mit maximaler eingestellter Leistung auf den Benutzer einzuwirken.

Sobald das Schlüsselwort erkannt wird, veranlasst die Auswerteeinheit die Steuerung 38 dazu, die mit einer Geräuschentwicklung verbundenen Betriebsparameter herabzusetzen, damit das Mikrofon 51 bei der Erfassung der Spracheingabe nicht durch die Geräusche gestört wird. Die erfolgreich identifizierte Spracheingabe quittiert die Auswerteeinheit durch ein akustisches Signal, das signalisiert, dass der Sprachbefehl verstanden wurde.

Durch die Spracheingabe ist es möglich, die Vorrichtung 10 ohne Einsatz der Augen auch im laufenden Betrieb auf die Bedürfnisse der Benutzer einzustellen. Insbesondere ist es nicht erforderlich, die Anordnungen zum Einwirken mit UV-Licht bei der Veränderung der Parameter auszuschalten.

Die Erfindung ist vorstehend anhand eines Ausführungsbeispiels beschrieben worden, bei dem die Vorrichtung nach Art einer Sonnenbank ausgebildet ist. Es versteht sich, dass die Kommunikation mit Sprache in der beschriebenen Weise auch bei anderen Vorrichtungen zum Einwirken auf Teile eines Körpers vorgesehen sein kann, zum Beispiel bei Wellnessgeräten, insbesondere bei Massageliegen.

Die Erfindung ist vorstehend anhand eines Ausführungsbeispiels beschrieben worden, bei dem die medizinisch-kosmetische Strahlung im Wesentlichen UV-Strahlung der Bereiche UV A und UV B umfasst. Es versteht sich, dass auch Strahlung der weiteren medizinisch-kosmetische Strahlungsspektren zur Bestrahlung der Haut oder unterhalb der Haut liegender Bereiche eingesetzt werden kann.

Die Erfindung ist vorstehend anhand eines Ausführungsbeispiels beschrieben worden, bei dem die medizinisch-kosmetische Strahlung durch Einsatz von Strahlröhren 22, 32 erzeugte UV-Strahlung ist. Es versteht sich, dass UV-Strahlung im Speziellen und medizinisch-kosmetische Strahlung im Allgemeinen auch auf andere Weise als durch den Einsatz von Strahlröhren erzeugt werden können, z.B. durch LEDs oder Halogenlampen.

Die Erfindung ist vorstehend anhand eines Ausführungsbeispiels beschrieben worden, bei dem die Vorrichtung 10 mit einem horizontal ausgerichteten Unterteil 30 mit einer Liegefläche 34 und einem herabschwenkbaren Oberteil 20 ausgebildet ist. Es versteht sich, dass die Vorrichtung auch als Stehbräuner ausgebildet sein kann, bei dem die Gehäuseteile, in denen die Mittel zum Einwirken untergebracht sind, vertikal aufgestellt sind, und bei dem das schwenkbare Gehäuseteil in der Art einer Tür für den Zutritt in den in Gestalt einer vertikalen Röhre oder eines vertikalen Tunnels ausgebildeten Innenraum vorgesehen ist. Das Mikrofon ist in diesem Fall zweckmäßig an dem stationären Gehäuseteil angeordnet, und zwar vorzugsweise in einem Bereich oberhalb desjenigen Abschnitts des Gehäuses, dem der Benutzer während der Einwirkung zugekehrt ist.

## Patentansprüche

1. Vorrichtung zum Einwirken auf zumindest Teile eines Körpers eines Benutzers mit medizinisch-kosmetischer Strahlung und/oder durch mechanische Beeinflussung, umfassend
Mittel zum Einwirken (22, 23, 32) auf den menschlichen Körper, die einstellbare Betriebsparameter umfassen, und
eine mit den Mitteln zum Einwirken (22, 23, 32) verbundene Steuerung (38), die die Betriebsparameter einstellt,
und wobei zumindest ein Mikrofon (51) vorgesehen ist, durch das Spracheingaben des Benutzers zur Steuerung der Betriebsparameter erfassbar sind, und
dass das Mikrofon (51) mit einer Auswerteeinheit verbunden ist, die von dem Mikrofon erfasste Spracheingaben in Steuerbefehle für die Mittel zum Einwirken (22, 23, 32) umwandelt, und ein Lautsprecher vorgesehen ist, und dass die Auswerteeinheit erfolgreich erkannte Spracheingaben mit einem akustischen Signal bestätigt, und
**dadurch gekennzeichnet, dass**
die Vorrichtung ausgestaltet ist um über Lautsprecher der Vorrichtung wiedergegebene akustische Signale über einen separaten Eingang der Auswerteeinheit zuzuführen und diese zu invertieren, so dass zufällig durch das Mikrofon erfassten Geräusche ausdrücklich nicht als Steuerbefehl verstanden werden.

2. Vorrichtung nach Anspruch 1, **dadurch gekennzeichnet, dass** die Auswerteeinheit eine Rechnereinheit mit einer Spracherkennungseinrichtung umfasst, die die durch das Mikrofon (51) erfassten Spracheingaben analysiert und die Steuerbefehle an die Steuerung (38) übergibt.

3. Vorrichtung nach einem der vorhergehenden Ansprüche, **dadurch gekennzeichnet, dass** die Auswerteeinheit durch Spracheingabe eines Schlüsselwortes aktivierbar ist.

4. Vorrichtung nach einem der vorhergehenden Ansprüche, **dadurch gekennzeichnet, dass** die Auswerteeinheit durch Berühren eines berührungsempfindlichen Bildschirms (40) aktivierbar ist.

5. Vorrichtung nach einem der vorhergehenden Ansprüche, **dadurch gekennzeichnet, dass** eine Taste (52) neben dem Mikrofon (51) vorgesehen ist, und dass die Auswerteeinheit durch Betätigen der Taste (52) aktivierbar ist.

6. Vorrichtung nach einem der vorhergehenden Ansprüche, **dadurch gekennzeichnet, dass** ein Display und ein Lautsprecher vorgesehen sind, und dass über das Display und den Lautsprecher optische und/oder akustische Erläuterungen der Sprachbefehle anzeigbar sind.

7. Vorrichtung nach einem der vorhergehenden Ansprüche, **dadurch gekennzeichnet, dass** bei Erfassung einer Spracheingabe mit einer Lautstärkeentwicklung verbundene Betriebsparameter durch die Steuerung (38) abgesenkt werden, und dass eine voreinstellbare Zeit nach Ende einer Eingabe die Absenkung rückgängig gemacht wird.

8. Vorrichtung nach einem der vorhergehenden Ansprüche, **dadurch gekennzeichnet, dass** die Betriebsparameter ausgewählt sind aus der Gruppe umfassend Gesichtslampen (23), Körperlampen (22, 32), LEDs, Körperlüftung, Gesichtslüftung, Temperatur, Feuchtigkeitszugabe, Musikwiedergabe, Lautstärke, Aromazugabe, Rotlicht, Blaulicht, Wasserstrahlstärke, Massageintensität, Massageprogramm, Lichtprogramm.

9. Vorrichtung nach einem der vorhergehenden Ansprüche, **dadurch gekennzeichnet, dass** zusätzlich zu der Bezeichnung des Betriebsparameters eine Parametergröße vorgebbar ist, die ausgewählt ist aus der Gruppe umfassend Plus, Minus, Maximum, Minimum, Standard, Ein, Aus.

10. Vorrichtung nach einem der vorhergehenden Ansprüche, **dadurch gekennzeichnet, dass** die Körperteile ausgewählt sind aus der Gruppe umfassend die Haut, das Bindegewebe, die Muskulatur, die Knochen.

11. Vorrichtung nach einem der vorhergehenden Ansprüche, **dadurch gekennzeichnet, dass** Sprachparameter des Benutzers auf einer Speichereinheit gespeichert sind und nach Einlesen der Daten zur Einstellung der Auswerteeinheit einsetzbar sind.

12. Vorrichtung nach einem der vorhergehenden Ansprüche, **dadurch gekennzeichnet, dass** die Steuerung (38) auch andere als die Mittel zum Einwirken auf den menschlichen Körper einstellt.

13. Vorrichtung nach einem der vorhergehenden Ansprüche, **dadurch gekennzeichnet, dass** die Vorrichtung als Hydromassagevorrichtung mit einer membranartigen Liegefläche für den Benutzer ausgebildet ist, umfassend zumindest eine unterhalb der Liegefläche aktivierbare Massagedüse, die einen Massagewasserstrahl gegen die membranartige Liegefläche zum Massieren des darauf liegenden Benutzers richten kann.

14. Vorrichtung nach einem der vorhergehenden Ansprüche, **dadurch gekennzeichnet, dass** die Mittel zum Einwirken ausgewählt sind aus der Gruppe umfassend UV-Licht emittierende Leuchtstoffröhren, sichtbares Licht emittierende Leuchtstoffröhren, UV-Licht emittierende LEDs, sichtbares Licht emittierende LEDs, UV-Licht emittierende Hochdrucklampen.

15. Vorrichtung nach einem der vorhergehenden Ansprüche, **dadurch gekennzeichnet, dass** ein tragbares Mobilgerät mit einem Mikrofon an die Vorrichtung anschließbar ist, und dass das Mikrofon des Mobilgeräts die Spracheingaben des Benutzers erfasst.

16. Vorrichtung nach einem der Ansprüche 1 bis 14, **dadurch gekennzeichnet, dass** die Mittel zum Einwirken (22, 23, 32) in einem selbsttragenden Gehäuse (24, 39) untergebracht sind, an dem das Mikrofon (51) angeordnet ist.

## Claims

1. A device for acting on at least parts of the body of a user, in particular with medical-cosmetic radiation and/or by mechanical influencing, comprising
means for acting (22, 23, 32) on the human body, which comprise adjustable operating parameters, and
a controller (38) connected to the means for acting (22, 23, 32), which sets the operating parameters,
and whereby at least one microphone (51) is provided, by which speech inputs of the user for controlling the operating parameters can be captured, and
in that the microphone (51) is connected to an evaluation unit which converts speech inputs captured by the microphone into control commands for the means for acting (22, 23, 32), and a loudspeaker is provided, and in that the evaluation unit confirms successfully recognized speech inputs with an acoustic signal, and
**characterized in that**
the device is laid out to conduct acoustic signals emitted by loudspeakers of the device via a sperate input to the evaluation unit and to invert these signals, such that sounds incidentally captured by the microphone are explicitly not read as control commands.

2. The device as claimed in claim 1, **characterized in that** the evaluation unit comprises a processor unit having a speech recognition unit, which analyzes the speech inputs captured by the microphone (51) and transfers the control commands to the controller (38).

3. The device as claimed in any one of the preceding claims, **characterized in that** the evaluation unit is activatable by speech input of a keyword.

4. The device as claimed in any one of the preceding claims, **characterized in that** the evaluation unit is activatable by touching a touch-sensitive display screen (40).

5. The device as claimed in any one of the preceding claims, **characterized in that** a button (52) is provided next to the microphone (51), and **in that** the evaluation unit is activatable by actuating the button (52).

6. The device as claimed in any one of the preceding claims, **characterized in that** a display and a loudspeaker are provided, and **in that** optical and/or acoustic explanations of the speech commands are displayable via the display and the loudspeaker.

7. The device as claimed in any one of the preceding claims, **characterized in that**, upon capture of a speech input, operating parameters linked to a volume development are lowered by the controller (38), and **in that** the lowering is reversed a pre-settable time after the end of an input.

8. The device as claimed in any one of the preceding claims, **characterized in that** the operating parameters are selected from the group comprising face lamps (23), body lamps (22, 32), LEDs, body ventilation, face ventilation, temperature, moisture feed, music playback, volume, aroma feed, red light, blue light, waterjet strength, massage intensity, massage program, light program.

9. The device as claimed in any one of the preceding claims, **characterized in that**, in addition to the designation of the operating parameter, a parameter variable is predefinable which is selected from the group comprising plus, minus, maximum, minimum, standard, on, off.

10. The device as claimed in any one of the preceding claims, **characterized in that** the body parts are selected from the group comprising the skin, the connective tissue, the musculature, the bones.

11. The device as claimed in any one of the preceding claims, **characterized in that** speech parameters of the user are stored on a memory unit and after reading in the data are usable for setting the evaluation unit.

12. The device as claimed in any one of the preceding claims, **characterized in that** the controller (38) also sets means other than the means for acting on the human body.

13. The device as claimed in any one of the preceding claims, **characterized in that** the device is designed as a hydromassage device with a membrane-like recumbent surface for the user, comprising at least one activatable massage nozzle underneath the recumbent surface, which can direct a massage waterjet against the membrane-like recumbent surface to massage the user lying thereon.

14. The device as claimed in any one of the preceding claims, **characterized in that** the means for acting are selected from the group comprising fluorescent tubes emitting UV light, fluorescent tubes emitting visible light, LEDs emitting UV light, LEDs emitting visible light, high-pressure lamps emitting UV light.

15. The device as claimed in any one of the preceding claims, **characterized in that** a portable mobile terminal having a microphone is connectable to the device, and **in that** the microphone of the mobile device captures the speech inputs of the user.

16. The device as claimed in any one of claims 1 to 15, **characterized in that** the means for acting (22, 23, 32) are housed in a self-supporting housing (24, 39), on which the microphone (51) is arranged.

## Revendications

1. Dispositif destiné à agir sur au moins des parties du corps d'un utilisateur, par rayonnement médico-cosmétique et/ou par influence mécanique, comprenant
des moyens d'action (22, 23, 32) sur le corps humain, qui comprennent des paramètres de fonctionnement réglables, et
une commande (38) reliée aux moyens d'action (22, 23, 32) qui règle les paramètres de fonctionnement,
et dans lequel au moins un microphone (51) est prévu par lequel des entrées vocales de l'utilisateur peuvent être détectées pour la commande des paramètres de fonctionnement, et
en ce que le microphone (51) est relié à une unité d'évaluation qui convertit les entrées vocales détectées par le microphone en instructions de commande pour les moyens d'action (22, 23, 32), et un haut-parleur est prévu, et en ce que l'unité d'évaluation confirme les entrées vocales correctement reconnues par un signal acoustique, et
**caractérisé en ce que**
le dispositif est conçu pour amener à l'unité d'évaluation, par l'intermédiaire d'une entrée séparée, des signaux acoustiques reproduits par le haut-parleur du dispositif et pour les inverser, de sorte que les bruits détectés de manière aléatoire par le microphone ne sont pas expressément compris comme une instruction de commande.

2. Dispositif selon la revendication 1, **caractérisé en ce que** l'unité d'évaluation comprend une unité informatique dotée d'un dispositif de reconnaissance vocale, qui analyse les entrées vocales détectées par le microphone (51) et transfère les instructions de commande à la commande (38).

3. Dispositif selon l'une des revendications précédentes, **caractérisé en ce que** l'unité d'évaluation peut être activée par entrée vocale d'un mot-clé.

4. Dispositif selon l'une des revendications précédentes, **caractérisé en ce que** l'unité d'évaluation peut être activée par un toucher sur un écran tactile (40).

5. Dispositif selon l'une des revendications précédentes, **caractérisé en ce qu'**un bouton (52) est prévu à côté du microphone (51), et **en ce que** l'unité d'évaluation peut être activée en appuyant sur le bouton (52).

6. Dispositif selon l'une des revendications précédentes, **caractérisé en ce qu'**un dispositif d'affichage et un haut-parleur sont prévus, et **en ce que** des explications visuelles et/ou acoustiques des commandes vocales peuvent être visualisées par l'intermédiaire du dispositif d'affichage et du haut-parleur.

7. Dispositif selon l'une des revendications précédentes, **caractérisé en ce que**, lorsqu'une entrée vocale est détectée, les paramètres de fonctionnement associés à une évolution du volume sont abaissés par la commande (38), et **en ce que** la réduction est inversée un temps prédéfini après la fin d'une entrée.

8. Dispositif selon l'une des revendications précédentes, **caractérisé en ce que** les paramètres de fonctionnement sont choisis dans le groupe comprenant les lampes faciales (23), les lampes corporelles (22, 32), les DEL, la ventilation corporelle, la ventilation faciale, la température, l'apport d'humidité, la lecture de musique, le volume, l'ajout d'arômes, la lumière rouge, la lumière bleue, la force du jet d'eau, l'intensité de massage, le programme de massage, le programme lumineux.

9. Dispositif selon l'une des revendications précédentes, **caractérisé en ce que**, en plus de la désignation des paramètre de fonctionnement, une grandeur de paramètre peut être spécifiée, qui est choisie dans le groupe comprenant plus, moins, maximum, minimum, standard, activé, désactivé.

10. Dispositif selon l'une des revendications précédentes, **caractérisé en ce que** les parties du corps sont choisies dans le groupe comprenant la peau, le tissu conjonctif, les muscles et les os.

11. Dispositif selon l'une des revendications précédentes, **caractérisé en ce que** les paramètres linguistiques de l'utilisateur sont stockés dans une unité de mémoire et peuvent être utilisés pour paramétrer l'unité d'évaluation après la lecture des données.

12. Dispositif selon l'une des revendications précédentes, **caractérisé en ce que** la commande (38) commande également des moyens autres que les moyens d'action sur le corps humain.

13. Dispositif selon l'une des revendications précédentes, le dispositif étant **caractérisé en ce qu'**il est conçu comme un appareil d'hydromassage avec une surface de couchage en forme de membrane pour l'utilisateur, comprenant au moins une buse de massage activable sous la surface de couchage et pouvant diriger un jet d'eau de massage contre la surface de couchage en forme de membrane pour masser l'utilisateur qui est allongé dessus.

14. Dispositif selon l'une des revendications précédentes, **caractérisé en ce que** les moyens d'action sont choisis dans le groupe comprenant les tubes fluorescents émettant de la lumière UV, les tubes fluorescents émettant de la lumière visible, les DEL émettant de la lumière UV, les DEL émettant de la lumière visible, les lampes haute pression émettant de la lumière UV.

15. Dispositif selon l'une des revendications précédentes, **caractérisé en ce qu'**un appareil mobile portable doté d'un microphone peut être connecté à l'appareil, et **en ce que** le microphone de l'appareil mobile détecte les entrées vocales de l'utilisateur.

16. Dispositif selon l'une des revendications 1 à 14, **caractérisé en ce que** les moyens d'action (22, 23, 32) sont logés dans un bâti autonome (24, 39) sur lequel est agencé le microphone (51).
